# EUROPEAN PATENT SPECIFICATION

(11) **EP 1 458 727 B1**
(45) Date of publication and mention of the grant of the patent: **31.08.2005**
(21) Application number: 02791870.5
(22) Date of filing: 19.12.2002
(51) Int. Cl.: C07D 501/00

(54) **PROCESS FOR THE STABILIZATION OF AN ACIDIC AQUEOUS PHASE COMPRISING CEPHRADINE**
PROZESS ZUR STABILISIERUNG EINER CEPHRADIN ENTHALTENDEN SAUREN WÄSSRIGEN PHASE
PROCEDE DE STABILISATION D'UNE PHASE AQUEUSE ACIDE COMPRENANT DE LA CEPHRADINE

(30) Priority: 27.12.2001 EP 01000793
(43) Date of publication of application: 22.09.2004
(73) Proprietor: DSM IP Assets B.V., 6411 TE Heerlen (NL)
(72) Inventor: ROY, Peter, Daniel, NL-5941 EA Velden (NL); LENHARDT, Carlos, Enrique, E-08328 Alella (Barcelona) (ES); KORTOOMS, Theodorus, Petrus, Maria, Antonius, NL-5913 AS Venlo (NL); STOELWINDER, Johannes, NL-6601 CW Wychen (NL)
(74) Representative: Elkenbracht, Johan Christiaan
(86) International application number: PCT/EP2002/014820
(87) International publication number: WO 2003/055892

(56) References cited:
- WO-A-91/00865
- US-A- 3 912 726

## Description

The invention relates to a process for the stabilization of an acidic aqueous phase comprising cephradine, HCl, water and dichloromethane in solution. The invention also relates to the stabilization of an acidic aqueous phase comprising cephradine, HCl, water and dichloromethane in solution, obtained in a chemical process for the production of cephradine.

In a chemicarprocess for the production of cephradine, the amino-group in 7-aminodesacetoxy cephalosporanic acid (7-ADCA) is acylated with a dihydrophenyl glycine side chain while dichloromethane is used as solvent. For example, D(-)-α-2,5-dihydrophenylglycine methyl sodium Dane salt and pivaloyl chloride in dichloromethane may be added to a solution of 7-ADCA and 1,8-diazabicydo-(5,4,0)-undec-7-ene (DBU) in dichloromethane. The resulting reaction mixture comprises cephradine with protected groups. The use of protected intermediates, such as cephradine with protecting groups, is characteristic for a chemical process for the production of cephalosporins, for example cephradine. To remove the protecting groups, water and HCl are added to the reaction mixture. The reaction mixture and added acidic solution form a two phase system, which is subsequently separated into two separate phases, an organic phase comprising mainly dichloromethane and pivalic acid with some residual water and an aqueous phase comprising water, cephradine and HCl in solution, as well as residual dichloromethane. Typically, the pH of the acidic aqueous solution, obtained through said chemical process for the production of cephradine and subsequent separation of the reaction mixture, is below 3.2. Subsequently, the formed cephradine is recovered from the aqueous phase by a pH shift; a complexing agent may be added. For example DMF or quinoline can be added to produce a solid cephradine complex, which is subsequently converted to cephradine.

A problem is that, especially on an industrial scale, uncontrolled crystallization of cephradine as the HCl salt can spontaneously occur in an acidic aqueous phase comprising cephradine, HCl, water and residual dichloromethane in solution. By uncontrolled crystallization is meant (in this context); crystallization that occurs at a time without a deliberate eternal stimulus for example a pH shift).Uncontrolled crystallization of cephradine as the HCl salt in a chemical process for the production of cephradine reduces both the yield and quality of the product cephradine. This is due to the need for filtration of any prematurely crystallized cephradine from the aqueous solution prior to the crystallization step. Filtration prolongs the overall product recovery and therefore leads to more degradation of the cephradine product and therefore to a lesser quality of the product (among others a reduction in shelf-life).

EP 433 428 discloses a process in which a cephradine salt is stabilized in an acidic aqueous phase by addition of dimethylformamide (DMF) to the reaction mixture comprising a cephradine salt and water. For the recovery of the cephradine product, subsequent to the addition of DMF, the aqueous phase is heated and a base is added to increase the pH. Upon addition of the base, crystallisation of cephradine.DMF solvate starts to occur. After dissolving the cephradine.DMF solvate in aqueous hydrochloric acid and adjusting the pH with a base, cephradine is obtained.

However, in this method to prevent the crystallization of cephradine as the HCl salt in an acidic aqueous phase, large amounts of DMF are needed. Large amounts of DMF are not advantageous, because this leads to the presence of residual DMF in the final product. Also, the use of these large amounts of DMF, makes this process less efficient for the recovery of DBU.

It is the object of the invention to provide an improved process for the stabilization of an acidic aqueous phase comprising cephradine, HCl, water and residual dichloromethane in solution. It is another object of the invention to provide a process for the production of cephradine, in which the chance that uncontrolled crystallization of cephradine as the HCl salt occurs is minimized. It is another object of the invention to provide a process for the production of cephradine in which the chance that uncontrolled crystallization of cephradine as the HCl salt occurs is minimized and in which the use of DMF is avoided. It is another object of the invention to provide a process for the production of cephradine in which the chance that uncontrolled crystallization of cephradine.HCl occurs is minimized and in which the use of DMF is avoided and in which DBU can be more efficiently recovered.

It has surprisingly been found that by subjecting an acidic aqueous phase, comprising cephradine, HCl, water and residual dichloromethane in solution, to a vacuum distillation, a stable aqueous phase is obtained in which the chance that uncontrolled crystallization of cephradine as the HCl salt occurs, is minimized. An added advantage of the invention is that the quality of cephradine is improved with respect to containing less dichloromethane in the final product.

The term 'residual dichloromethane' means that there is more than 1.0% w/w dichloromethane present in the acidic aqueous phase. Typically, the content of residual dichloromethane in the acidic aqueous phase obtained after the separation of an organic phase and an aqueous phase produced in a chemical process for the production of cephradine is in the range of 3 to 7% w/w.

The invention therefore relates to a process for the stabilization of an acidic aqueous phase comprising cephradine, HCl, water and residual dichloromethane in solution, characterized in that the aqueous phase is subjected to a vacuum distillation. The invention can suitably be applied in a chemical process for the production of cephradine, wherein after the separation of an organic phase and an aqueous phase, an acidic aqueous phase comprising cephradine, HCl, water and residual dichloromethane in solution is produced. Therefore, the invention also relates to a process for the production of cephradine, in which the acidic aqueous phase obtained after the separation of an organic phase and an aqueous phase produced in a chemical process for the production of cephradine and which comprises cephradine, HCl, water and residual dichloromethane in solution, is subjected to a vacuum distillation. Thereby, a process has been found, in which an acidic aqueous phase comprising cephradine, HCl, water and residual dichloromethane in solution is stabilized without the use of DMF and wherein the chance that uncontrolled crystallization of cephradine as the HCl salt occurs is minimized. This has the advantage that cephradine can be obtained in a higher purity and therefore, less isolation and subsequent purification steps, for example recrystallization steps, are needed.

Cephradine may be prepared in a chemical process comprising: (i) acylating the amino-group in 7-aminodesacetoxy cephalosporanic acid (7-ADCA) with a dihydrophenyl glycine side chain in the presence of dichloromethane, resulting in a reaction mixture; (ii) adding HCl and water to the reaction mixture, resulting in a two phase system, said two phase system comprising an organic phase and an acidic aqueous phase, said acidic aqueous phase comprising water, cephradine, HCl and dichloromethane. The acidic aqueous phase and organic phase may be separated in any suitable way, for instance phase separation. Preferably, said acylating is carried out in the presence of pivaloyl chloride and/or 1,8-diazabicyclo-(5,4,0)-undec-7-ene. Preferably, the process comprises acylating said 7-aminodesacetoxy cephalosporanic acid using D(-)-α-2,5-dihydrophenylglycine methyl sodium Dane salt. In a preferred embodiment, the process comprises adding D(-)-α-2,5-dihydrophenylglycine methyl sodium Dane salt and pivaloyl chloride in dichloromethane to a solution of 7-ADCA and 1,8-diazabicyclo-(5,4,0)-undec-7-ene (DBU) in dichloromethane.

Preferably, the pH of the acidic aqueous phase is below 3.2.

The vacuum distillation is preferably carried out as a continuous distillation. The temperature and the pressure of the vacuum distillation may vary between wide ranges. Preferably, the vacuum distillation in the process according to the invention is carried out at a temperature between 0 and 40°C, preferably between 10 and 40°C, more preferably between 30 and 40°C, most preferably between 34 and 38°C. Preferably, the vacuum distillation is started at a temperature between 34-38°C. Preferably, the vacuum distillation is carried out at a pressure below 450 mm Hg. At the start of the vacuum distillation, the pressure is preferably between 450 and 350 mm Hg.

The acidic aqueous phase to be subjected to the vacuum distillation comprises dichloromethane, for instance more than 1.0% w/w dichloromethane, for instance between 3 to 7% w/w dichloromethane.

The vacuum distillation may be carried out in any suitable way, such as to separate dichloromethane from the acidic aqueous phase. As a result the concentration of dichloromethane in the acidic aqueous phase is decreased. Surprisingly, the separation of dichloromethane from an acidic aqueous phase comprising cephradine, HCl, water and dichloromethane is found to result in an increased stability of the acidic aqueous solution. Accordingly, a process is also provided comprising separating dichloromethane from an acidic aqueous phase, said acidic aqueous phase comprising cephradine, HCl, water and dichloromethane. The concentration dichloromethane in the acidic aqueous phase prior to said separating may for instance be more than 1.0% w/w dichloromethane, for instance between 3 to 7% w/w dichloromethane. Preferably, the separation is carried out such as to obtain a concentration dichloromethane in the acidic aqueous phase of less than 1.0% w/w, preferably less than 0.8% w/w. The vacuum distillation is preferably continued until the quantity of dichloromethane remaining in the aqueous phase after vacuum distillation is less than 1.0% w/w, preferably less than 0.8 % w/w. Decreasing the concentration of dichloromethane below these values further improves the stability. Accordingly, the invention also provides an acidic aqueous solution comprising cephradine, HCl, water and dichloromethane, the concentration dichloromethane in said acidic aqueous solution being less than 1.0% w/w, preferably less than 0.8% w/w. The amount of dichloromethane present in the aqueous phase can be analyzed with gas chromatography (GC). Alternatively, the end-point of the vacuum distillation can be determined by the pressure. The vacuum distillation is preferably continued until the pressure of the vacuum distillation is equal to or less than 150 mm Hg.

Cephradine can be isolated from the stabilized acidic aqueous solution by changing the pH to a pH suitable for crystallization. The temperature at which this crystallization takes place is preferably between 40 and 70°C, more preferably between 48 and 62°C.

The invention is illustrated by way of the following examples. However, these examples are not meant to restrict the invention.

### Examples

### Example I. Including the introduction of a vacuum distillation step resulting in a stable aqueous solution of cephradine and hydrochloric acid.

### 1) MIXED ANHYDRIDE PREPARATION

A suspension of N-methylacetamide (21.7 grs; 0.30 moles), D(-)-α-2,5-dihydrophenylglycine methyl sodium Dane salt (269.9 grs; 0.987 moles), gamma-picoline (0.055 ml; 0.6 mmoles) in dichloromethane (980 ml) was cooled to -20°C, treated with pivaloyl chloride (119.7 grs; 0.991 moles) at -10°C during10 minutes, and cooled to -35°C.
This system was called preparation A

### 2) 7-ADCA SOLUTION

Dichloromethane (679 ml) was cooled to 5°C and 7 ADCA (175 grs; 0.817 moles) was loaded. Then diazabicyclo (5,4,0) undec-7-ene (140 grs; 0.920 moles) was added with stirring to obtain complete solution.
This system was called preparation B

### 3) COUPLING (ACYLATION)

Preparation B was added into preparation A, keeping the temperature at-35°C. The resulting reaction mixture C was stirred for 3 hours.

### 4) HYDROLYSIS

Water (980 ml) and hydrochloric acid 35% (210 grs; 2.0 moles) were added to reaction mixture C and the temperature was brought up to 15°C. The pH was in the range of 1.5 to 2.0. Then, after stirring for 15 minutes, the layers were separated.

### 5) VACUUM DISTILLATION

The aqueous (upper) layer obtained in step 4 was immediately warmed up to 38°C and simultaneously distilled under vacuum conditions (initial pressure of about 360 mmHg; final pressure 150 mmHg) to remove the residual dichloromethane. The vacuum distillation was stopped when the GC (gas chromatography) showed a dichloromethane content of 0.7% w/w. A stable (with respect to uncontrolled crystallization of cephradine hydrochloric salt), clear solution, containing cephradine was obtained.

### 6) ISOLATION

The obtained solution was warmed up to 60 °C and cephradine was crystallized by adding triethylamine until pH=5. After filtration, 220g (0.60 moles; 73% yield) of cephradine (99% assay on dry basis) were obtained. The purity of the thus obtained cephradine was within the specifications of the Chinese Pharmacopeia.

### Comparative example A. Excluding a vacuum distillation step resulting in a potentially unstable aqueous solution of cephradine and hydrochloric acid.

Steps 1-4 were performed according to example I. Step 5 was not performed and instead the aqueous (upper) layer obtained in step 4 was kept during 10 minutes at 15°C with stirring. After this period of time, a slurry of crystals was obtained. The crystals were filtered, washed first with 100 ml of isopropyl alcohol followed by 300 ml of n-hexane and dried. The product was identified as cephradine as the hydrochloric acid salt.

## Claims

1. Process for the stabilization of an acidic aqueous phase comprising cephradine, HCl, water and dichloromethane, said process comprising subjecting said acidic aqueous phase to a vacuum distillation.

2. Process for the production of cephradine, wherein the acidic aqueous phase obtained after the separation of an organic phase and an aqueous phase produced in a chemical process for the production of cephradine and which comprises cephradine, HCl, water and dichloromethane, is subjected to a vacuum distillation.

3. Process according to claim 1 or 2, wherein the vacuum distillation is carried out as a continuous vacuum distillation.

4. Process according to any of claims 1-3, wherein the vacuum distillation is carried out at a temperature between 0 and 40 °C.

5. Process according to any of claims 1-4, wherein the vacuum distillation is started at a pressure between 450 and 350 mm Hg.

6. Process according to any one of claims 1-5, wherein the acidic aqueous phase comprises more than 1.0% w/w dichloromethane.

7. Process according to claim 6, wherein the acidic aqueous phase comprises between 3 to 7% w/w dichloromethane.

8. Process according to claim 6 or claim 7, wherein the vacuum distillation is continued until the quantity of dichloromethane remaining in the aqueous solution is less than 1.0% w/w.

9. Process according to claim 8, wherein the vacuum distillation is continued until the quantity of dichloromethane remaining in the aqueous solution is less than 0.8 % w/w.

10. Process according to any of claims 1-9, wherein the vacuum distillation is continued until the pressure of the distillation is equal to or less than 150 mm Hg.

11. Process according to any of claims 1-10, wherein the stabilized acidic aqueous phase obtained by the process according to any of claims 1-9 is further subjected to an isolation step in which cephradine is crystallized by changing the pH to a pH where cephradine crystallizes.

12. Process according to claim 11, wherein the isolation step takes place at a temperature in the range from 48 to 62°C.

13. Acidic aqueous solution comprising, cephradine, HCl, water and dichloromethane, in which solution the concentration dichloromethane is less than 1.0% w/w, preferably less than 0.8% w/w.

14. Process comprising subjected the solution according to claim 13 to an isolation step in which cephradine is crystallized by changing the pH to a pH where cephradine crystallizes.

15. Process according to claim 14, wherein the isolation step takes place at a temperature in the range from 48 to 62°C.

## Patentansprüche

1. Verfahren zum Stabilisieren einer sauren wäßrigen Phase, enthaltend Cefradin, HCl, Wasser und Dichlormethan, wobei das Verfahren eine Stufe beinhaltet, in der die saure wäßrige Phase einer Vakuumdestillation unterworfen wird.

2. Verfahren zum Herstellen von Cefradin, worin die saure wäßrige Phase, welche nach dem Trennen einer organischen Phase und einer wäßrigen Phase erhalten worden ist, die in einem chemischen Verfahren zum Herstellen von Cefradin gebildet worden ist und Cefradin, HCl, Wasser und Dichlormethan enthält, einer Vakuumdestillation unterworfen wird.

3. Verfahren nach Anspruch 1 oder 2, worin die Vakuumdestillation als kontinuierliche Vakuumdestillation durchgeführt wird.

4. Verfahren nach einem der Ansprüche 1 bis 3, worin die Vakuumdestillation bei einer Temperatur zwischen 0 und 40 °C durchgeführt wird.

5. Verfahren nach einem der Ansprüche 1 bis 4, worin die Vakuumdestillation bei einem Druck zwischen 450 und 350 mm Hg begonnen wird.

6. Verfahren nach einem der Ansprüche 1 bis 5, worin die saure wäßrige Phase mehr als 1,0 % Gewicht/Gewicht Dichlormethan enthält.

7. Verfahren nach Anspruch 6, worin die saure wäßrige Phase zwischen 3 und 7 % Gewicht/Gewicht Dichlormethan enthält.

8. Verfahren nach Anspruch 6 oder 7, worin die Vakuumdestillation fortgesetzt wird, bis in der wäßrigen Lösung zurückbleibende Menge an Dichlormethan weniger als 1,0 % Gewicht/Gewicht beträgt.

9. Verfahren nach Anspruch 8, worin die Vakuumdestillation fortgesetzt wird, bis die in der wäßrigen Lösung zurückbleibende Menge an Dichlormethan weniger als 0,8 % Gewicht/Gewicht beträgt.

10. Verfahren nach einem der Ansprüche 1 bis 9, worin die Vakuumdestillation fortgesetzt wird, bis der Destillationsdruck 150 mm Hg oder weniger beträgt.

11. Verfahren nach einem der Ansprüche 1 bis 10, worin die durch das Verfahren gemäß einem der Ansprüche 1 bis 9 erhaltene stabilisierte saure wäßrige Phase ferner einer Isolierungsstufe unterworfen wird, in der Cefradin auskristallisiert wird durch Ändern des pH-Werts in einen pH-Wert, bei dem Cefradin auskristallisiert.

12. Verfahren nach Anspruch 11, worin die Isolationsstufe bei einer Temperatur im Bereich von 48 bis 62 °C durchgeführt wird.

13. Saure wäßrige Lösung, enthaltend Cefradin, HCl, Wasser und Dichlormethan, wobei in der Lösung die Konzentration von Dichlormethan weniger als 1,0 % Gewicht/Gewicht, vorzugsweise weniger als 0,8 % Gewicht/Gewicht, beträgt.

14. Verfahren, bei dem die Lösung gemäß Anspruch 13 einer Isolierungsstufe unterworfen wird, in der Cefradin auskristallisiert wird durch Ändern des pH-Werts in einen pH-Wert, bei dem Cefradin kristallisiert.

15. Verfahren nach Anspruch 14, worin die Isolierungsstufe bei einer Temperatur im Bereich von 48 bis 62 °C durchgeführt wird.

## Revendications

1. Procédé de stabilisation d'une phase aqueuse acide comprenant de la céphradine,HCl, de l'eau et du dichlorométhane, ledit procédé comprenant l'étape consistant à soumettre ladite phase aqueuse acide à une distillation sous vide.

2. Procédé de production de céphradine, dans lequel la phase aqueuse acide obtenue après la séparation d'une phase organique et d'une phase aqueuse produite lors d'un procédé chimique de production de céphradine et qui comprend de la céphradine,HCl, de l'eau et du dichlorométhane, est soumise à une distillation sous vide.

3. Procédé selon les revendications 1 ou 2, dans lequel la distillation sous vide réalisée est une distillation sous vide en continu.

4. Procédé selon l'une quelconque des revendications 1 à 3, dans lequel la distillation sous vide est réalisée à une température comprise entre 0°C et 40°C.

5. Procédé selon l'une quelconque des revendications 1 à 4, dans lequel la distillation sous vide commence à une pression comprise entre 450 et 350 mm Hg.

6. Procédé selon l'une quelconque des revendications 1 à 5, dans lequel la phase aqueuse acide comprend plus de 1,0% p/p de dichlorométhane.

7. Procédé selon la revendication 6, dans lequel la phase aqueuse acide comprend entre 3% et 7% p/p de dichlorométhane.

8. Procédé selon les revendications 6 ou 7, dans lequel la distillation sous vide se poursuit jusqu'à ce que la quantité de dichlorométhane restant dans la solution aqueuse soit inférieure à 1,0% p/p.

9. Procédé selon la revendication 8, dans lequel la distillation sous vide se poursuit jusqu'à ce que la quantité de dichlorométhane restant dans la solution aqueuse soit inférieure à 0,8% p/p.

10. Procédé selon l'une quelconque des revendications 1 à 9, dans lequel la distillation sous vide se poursuit jusqu'à ce que la pression de la distillation soit inférieure ou égale à 150 mm Hg.

11. Procédé selon l'une quelconque des revendications 1 à 10, dans lequel la phase aqueuse acide stabilisée obtenue par le procédé selon l'une quelconque des revendications 1 à 9 est ensuite soumise à une étape d'isolation dans laquelle la céphradine cristallise en changeant le pH à un pH pour lequel la céphradine cristallise.

12. Procédé selon la revendication 11, dans lequel l'étape d'isolation est effectuée à une température dans la gamme de 48°C à 62°C.

13. Solution aqueuse acide comprenant de la céphradine,HCl, de l'eau et du dichlorométhane, dans laquelle la concentration en dichlorométhane est inférieure à 1,0% p/p, de préférence inférieure à 0,8% p/p.

14. Procédé comprenant l'étape consistant à soumettre la solution de la revendication 13 à une étape d'isolation dans laquelle la céphradine cristallise en changeant le pH à un pH pour lequel la céphradine cristallise.

15. Procédé selon la revendication 14, dans laquelle l'étape d'isolation est effectuée à une température dans la gamme de 48°C à 62°C.
